# EUROPEAN PATENT SPECIFICATION

(11) **EP 0 429 552 B1**
(45) Date of publication and mention of the grant of the patent: **27.11.1996**
(21) Application number: 89912592.6
(22) Date of filing: 27.10.1989
(51) Int. Cl.: A61N 1/00

(54) **IONTOPHORESIS ELECTRODE**
Iontophorese Elektrode
ELECTRODE D'IONTOPHORESE

(30) Priority: 28.10.1988 US 264239
(43) Date of publication of application: 05.06.1991
(73) Proprietor: ALZA CORPORATION, Palo Alto California 94303-0802 (US)
(72) Inventor: PHIPPS, Joseph, B., Plymouth, MN 55442 (US)
(74) Representative: Tomlinson, Kerry John
(86) International application number: US8904831
(87) International publication number: WO9004432

(56) References cited:
- DE-A- 3 703 321
- JP-A-60 203 270
- US-A- 3 994 302
- US-A- 4 336 811
- US-A- 4 416 274
- US-A- 4 640 689
- US-A- 4 720 334
- US-A- 4 722 726
- US-A- 4 731 049
- US-A- 4 744 787
- US-A- 4 747 819
- US-A- 4 752 285
- BIOMEDIZINISCHE TECHNIK. vol. 26, no. 3, March 1981, BERLIN DE pages 43 - 53; J.G. SCHINDLER ET AL.: 'Technologien für elektrochemische Festkontakt-Katheter- Sensoren und Teleskop-Katheter-Elektroden-Systheme zur Bestimmung von Ca++ , K+, Na+, Cl-, Harnstoff, pH, O2 und CO2'
- Journal of Pharmaceutical Sciences, Vol. 76, No. 3, March 1987, (SANDERSON ET AL), "Noninvasive Delivery of a Novel Inotropic Catecholamine: Iontophoretic Versus Intravenous in Dogs", pages 215-218.

## Description

### BACKGROUND OF THE INVENTION

This invention relates to methods and apparatus for transdermal medicament delivery and to improvements therein. More specifically, this invention relates to improved methods and apparatus for active (as opposed to passive) transdermal, ambulatory drug delivery. Yet more particularly, this invention relates to increasing the efficiency of iontophoresis devices and to improved methods of making and using such devices.

Recently, there has been a renewed interest in the technology of iontophoresis. Iontophoresis has been found to be useful in the transdermal administration or introduction of lidocaine hydrochloride, hydrocortisone, acetic acid, flouride, penicillin, dexamethasone sodium phosphate, and many other drugs. Perhaps the widest use of iontophoresis is the diagnosis of cystic fibrosis using pilocarpine nitrate iontophoresis.

In presently known iontophoresis devices, at least two electrodes are used. Both these electrodes are disposed so as to be in intimate electrical contact with some portion of the skin. The "active" electrode is the electrode from which the ionic drug is delivered into the body. The "indifferent" or ground electrode serves to close the electrical circuit through the body. A battery or other current source is coupled to the electrode to provide the electrical force to drive the drug into the body. For example, if the ionic substance to be driven into the body is positively charged, then the positive electrode (the anode) will be the active electrode and the negative electrode (the cathode) will serve to complete the circuit. If the ionic substance to be delivered is negatively charged, then the negative electrode will be the active electrode and the positive electrode will be the indifferent electrode. Of course, simultaneous delivery of drugs from both of the electrodes is also possible.

Generally, iontophoresis electrodes include a reservoir of the drug, typically compounded as a salt of the drug, for example a flouride or sulfate. These reservoirs may take the form of preformed gel bodies, such as disclosed in U.S. Patent No. 4,382,529 issued to Webster, solid adhesive bodies as disclosed in U.S. Patent No. 4,416,274, issued to Jacobson, or fluid reservoirs as disclosed in U.S. Patent No. 4,250,878, issued to Jacobsen. Electrical current is typically applied to the fluid reservoir by means of a current distributing member, which may take the form of a metal plate, a foil layer, a conductive screen, or a dispersion of conductive particles within the drug reservoir.

Typically, the current distributing member in iontophoresis electrodes has been constructed of an inert material, such as stainless steel or platinum. However, more recently use of sacrificial current distributing members which are oxidized or reduced themselves during delivery of the drug has been discussed. Use of sacrificial current distributing members can avoid the pH changes and other adverse effects associated with the hydrolysis of water which generally accompanies the use of inert current distributing members. Electrodes with sacrificial current distributing members are disclosed in U.S. Patent No. 4,744,787, issued to Phipps et al,

An alternative approach to avoiding the adverse effects associated with hydrolysis of water at the current distributing member is disclosed in the published PCT Patent Application No. WO 87/04936, published August 27, 1987, by Sanderson et al, corresponding to U.S. Patent No. 4,722,726. This electrode system is also described in the article "Noninvasive Delivery of a Novel Inotropic Catecholamine: Iontophoretic Versus Intravenous Infusion in Dogs" by Sanderson et al, published in the Journal of Pharmaceutical Sciences, Vol. 76, No. 3, March 1987, pp. 215-218. In this electrode system, an inert current distributing member is used and the electrode is divided into an upper chamber filled with a buffer and a lower chamber containing the ionic drug. The upper chamber is separated from the lower chamber by means of an ion selective membrane. As described, it is apparently intended that the buffer solution in the upper chamber mitigate the effects of hydrolysis of water, and that the ion selective membrane isolate the drug from the contents of the upper chamber.

In electrodes including fluid reservoirs, as disclosed in U.S. Patent No. 4,250,878 issued Jacobson, delivery of the drug typically takes place through a microporous membrane. Typically, such membranes are permeable based on size, and therefore must be permeable to any ion equal to or smaller than the drug ion intended to be delivered. In U.S. Patent No. 4,640,689, issued on February 3, 1987 to Sibalis, an iontophoresis electrode including a gel type drug reservoir provided with a semipermeable membrane is disclosed. This reference also suggests the use of an "ion selective retention gel" intermediate the drug reservoir and the semipermeable membrane. The ion to be retained by the gel is not discussed.

US-A-4752285 discloses the use of sacrificial electrodes in an iontophoresis bandage. A silver/silver chloride electrode is mentioned as an example. This document teaches that, by a suitable choice of electrode and medicament, extreme changes in pH can be avoided, leading to prevention of skin burns.

### SUMMARY OF THE INVENTION

In one aspect, the present invention provides an iontophoresis electrode comprising:
a conductive, current distributing member;
means for coupling said current distributing member to a source of electrical current; and
a reservoir containing an ionic or ionizable drug to be delivered;
characterised in that there is a layer of charge selective material applied to said current distributing member, intermediate said current distributing member and said reservoir, said material being selective for ions having a charge opposite to the charge of said drug when ionized.

The present invention relates to an improvement to iontophoresis electrodes. The invention is especially beneficial when embodied in iontophoresis electrodes of the type employing sacrificial cathodes or anodes which are oxidized or reduced, respectively, during iontophoretic drug delivery. The use of such sacrificial current distributing members avoids electrolysis of water, as the materials chosen for the current distributing members are oxidized or reduced at a lower voltage than required to hydrolyze water. For example, the positive electrode (anode) may be silver and the negative electrode (cathode) may be silver/silver chloride. The invention is also believed beneficial when embodied in iontophoresis electrodes employing inert current distributing members.

In conjunction with such electrodes, the present invention supplies an improvement in the form of a coating of a cation or anion selective material applied directly to the current distributing member. A cation selective material would be applied to the current distributing member in the cathode (negative electrode) and an anion selective material would be applied to the current distributing member in the anode (positive electrode). This coating will prevent the migration of ions produced during the oxidation or reduction of a sacrificial current distributing member into the drug reservoir. In addition, the charge selective material prevents direct contact between the current distributing member and the drug ions in the reservoir. This minimizes electrochemical oxidation or reduction of the drug, and is believed beneficial in preventing drug degradation.

### BRIEF DESCRIPTION OF THE DRAWINGS

Fig. 1 shows a sectional view through an electrode according to the present invention.

Fig. 2 shows a sectional view through a second embodiment of an electrode according to the present invention.

### DETAILED DESCRIPTION OF THE INVENTION

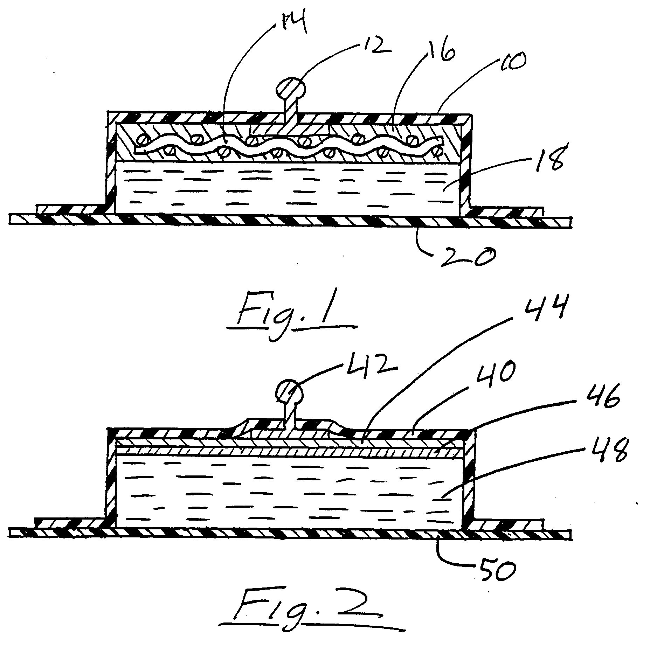

Fig. 1 shows a sectional view through an active iontophoresis electrode according to the present invention. The electrode is provided with a housing 10, which may be fabricated of an insulative plastic, such as polyvinyl chloride or polyethylene. An electrical snap connector 12 extends from the top side of housing 10, and is electrically coupled to a screen 14 which serves as the current distribution member. Reservoir 18 contains the drug to be delivered which typically is either ionic drug or is readily ionizable within the reservoir. Screen 14 is preferably fabricated of a material which is reduced or oxidized at an electrical potential less than that required to hydrolyze water. Suitable examples are, for example, silver for the positive electrodes (anodes) and silver/silver chloride for the negative electrodes (cathodes). Alternatively, screen 14 may be fabricated of an inert metal such as platinum or stainless steel.

Surrounding screen 14 is a layer of charge selective material 16 which is permeable to ions having a charge opposite that of the drug in reservoir 18. For example, if the electrode is a positive electrode used to deliver a positively charged drug, the layer of charge selective material 16 would be an anion selective material. Conversely, if the electrode of Fig. 1 is the negative electrode, used to deliver a negatively charged drug, the layer of charge selective material 16 would be a cation selective material.

Examples of anionic and cationic selective membranes are described in the article "Acrylic Ion-Transfer Polymers", by Ballestrasse et al, published in the Journal of the Electrochemical Society, November 1987, Vol. 134, No. 11, pages 2745-2749. An additional appropriate anion exchange membrane would be a copolymer of styrene and divinyl benzene reacted with trimethylamine to provide an anion exchange membrane (see "Principles of Polymer Systems", by F. Rodriguez, McGraw-Hill Book Co., 1979, pages 382-390). An additional appropriate cationic permeable membrane for use in conjunction with delivery of a negatively charged drug would be a sulfonated styrene polymer or a sulfonated fluorocarbon polymer, e.g. Nafion™ membranes, a product of DuPont.

The provision of coating 16 has several important benefits. First, it prevents interaction of the ionic drug and the current distribution member during storage. This is believed beneficial in extending the shelf life of iontophoresis electrodes and makes possible combinations of drugs and current distribution members which might otherwise not provide an appropriate shelf life.

In the context of shelf life, providing the layer of charge selective material 16 in direct contact with the current distribution member 14 is especially important. Although an ion selective materials as discussed above select for negatively charged ions, positively charged ions will diffuse through them, over time, given the presence of a concentration gradient across the material. For example, the above cited Sanderson references suggest construction of an iontophoresis electrode in two chambers, the upper chamber containing the current distribution member being filled with a buffer solution, the lower chamber being filled with a drug solution, and an anion selective membrane provided intermediate the upper and lower chambers. Diffusion of positive ions from both the upper buffer chamber and the lower drug chamber would occur across the anion selective membrane and would occur at a sufficient rate to severely limit the shelf life of such an electrode. This may require that the electrode be assembled shortly before use.

Because the electrode according to the present invention provides a layer of charge selective material 16 directly applied to the current distributing member, diffusion of drug ions across the material should not occur in appreciable amounts. This allows for the electrode to be constructed in advance, while maintaining an extended shelf life.

In embodiments employing a sacrificial current distribution member, the provision of an ion selective coating is particularly advantageous. In use, a source of electrical current will be coupled to snap connector 12, and thereby to screen 14. Typically, such power supplies are constant current power supplies, and the voltage differential between screen 14 and drug reservoir 18 will thereby be determined by the voltage differential required to reduce or oxidize the material of screen 14.

If the drug to be delivered is a positive drug, for example lithium, screen 14 would be fabricated of a readily oxidizable material such as silver, the layer of charge selective material 16 would take the form of an anionic selective material, and the drug would preferentially be compounded with a counter ion which reacts with ionic silver to form a neutrally charged compound. One example would be lithium chloride. When coupled to the power supply, screen 14 will be oxidized to produce silver ions. However, material 16 will subtantially reduce the migration of silver ions into the reservoir 18, where they might migrate in competition with the lithium ion. Instead, chloride ions will migrate across the layer of charge selective material 16 (in this case selective for anions), to form a silver chloride precipitate at the screen. This leaves lithium free to migrate with reduced competition from other positive ions.

If the drug to be delivered is a negatively charged ion, screen 14 would be fabricated of a readily reducible material, such as silver/silver chloride, the layer of charge selective material 16 would be a cationic selective material, and the drug in the reservoir would be compounded with a counter ion which forms a neutrally charged compound when combined with ionic chlorine. Examples of appropriate drug compounds would be copper or silver salicylate. In use, ionic silver in the silver chloride portion of screen 14 would be reduced, producing mobile chloride ions. A layer 16 of charge selective material in the form of cation selective material would substantially reduce migration of chloride ions into reservoir 18. Instead, positively charged copper or silver ions would migrate across material 16 to form a neutrally charged silver or copper chloride precipitate at screen 14. This leaves salicylate ion free to migrate with reduced competition from other negative ions.

This electrode construction provides significant additional advantages over prior art iontophoresis electrodes. For example, this construction reduces any toxic effects associated with the use of a silver current distribution member, and may make possible the use of materials such as lead, which would otherwise be counterindicated. In addition, it allows the use of materials for current distribution member 14 which in their ionized state might otherwise react with the ionic drug in reservoir 18 during use of the electrode.

As noted above, the invention may be practiced in conjunction with inert current distributing members. This approach is particularly valuable in conjunction with the delivery of drugs which take the form of weak acids or weak bases. In these electrodes, hydrolysis of water is deliberately induced, with the hydrolysis product combining with the drug as compounded to produce an ionic, mobile species. For example, a weakly acidic drug D may be placed in a drug reservoir including a platinum current distributing member, which functions as the anode of the iontophoresis system. Hydrolysis of water occurs at the anode, with excess hydrogen ions combining with the drug to produce a charged species DH⁺_{,} which is substantially the only charged species within the reservoir. Corresponding systems employing weakly basic drugs may also be produced. Such systems are described in more detail in U.S. Patents Nos. 5135477 and 5415628 by Untereker et al.

As manufactured, it is anticipated that the drug reservoir 18 will take the form of a solid or semisolid gel. In this case, the release liner 20 would typically be provided to seal the drug reservoir 18 against contamination and to prevent the gel in reservoir 18 from drying out over time. Alternative embodiments of the invention may employ fluid drug reservoirs surrounded by semipermeable membranes.

Preferably, drug reservoir 18 is free of ionic or readily ionizable material other than the drug to be delivered. For example, the matrix may take the form of a polar, nonionic gel, such as a polyvinyl alcohol gel or a gel as disclosed in EPO Patent No. 0 060 451, issued on September 17, 1986 to Lattin et al. This EPO patent is incorporated by reference herein in its entirety.

Fig. 2 is a sectional view of an alternative embodiment of an iontophoresis electrode according to the present invention. The electrode is provided with a housing 40, which may be fabricated of an insulative plastic, such as polyvinyl chloride or polyethylene. An electrical snap connector 42 extends from the top side of housing 40, and is electrically coupled to metallic foil 44 which serves as a current distribution member. Foil 44 may be fabricated of a material such as silver or silver chloride which is reduced or oxidized at an electrical potential less than required to hydrolize water, or may be an inert metal such as platinum or stainless steel. An ion selective material 46 is applied as a coating or layered directly over foil 40, and serves the same function as the layer of charge selective material 16, discussed in conjunction with Fig. 1. The iontophoretic drug for delivery is contained within reservoir 48, which will take the form of a solid or semisolid gel in the preferred embodiment. A release liner 50 is provided to seal the drug reservoir 48 against contamination and to prevent the reservoir 18 from drying out over time.

An electrode according to the present invention may also employ a charge selective ion permeable membrane at the interface of the drug reservoir and the skin, as disclosed in WO 90/04433 entitled "IONTOPHORESIS ELECTRODE" by Untereker et al. In such a case, the membrane applied between the reservoir and the skin would selectively pass ions having the charge of the ionic drug to be delivered.

## Claims

1. An iontophoresis electrode comprising:
a conductive, current distributing member (14);
means (12) for coupling said current distributing member to a source of electrical current; and
a reservoir (18) containing an ionic or ionizable drug to be delivered;
characterised in that there is a layer (16) of charge selective material applied to said current distributing member, intermediate said current distributing member and said reservoir, said material being selective for ions having a charge opposite to the charge of said drug when ionized.

2. An iontophoresis electrode as claimed in claim 1 characterised in that:
the current distributing member (14) is fabricated of a material which is readily oxidized or reduced at a voltage less than the voltage required to hydrolyze water; and
the reservoir (18) is coupled to said current distributing member and contains an ionic drug compounded with a counter-ion which reacts with said material of which said current distributing member is fabricated, after said material is oxidized or reduced, to produce a compound insoluble within said reservoir.

3. A method of fabricating an iontophoresis electrode comprising the steps of:
selecting an ionic or ionizable drug to be delivered;
including said drug within a reservoir through which said drug is permeable;
selecting a conductive current distributing member;
selecting a charge selective material selectively permeable to ions of the charge opposite to that of said drug when ionized; and
assembling said electrode by applying said charge selective material to said current distributing member and mounting said current distributing member to said reservoir such that said charge selective material is located intermediate said current distributing member and said reservoir.

4. A method of fabricating an iontophoresis electrode as claimed in claim 3 wherein:
the drug selected to be delivered is an ionic drug;
a sacrificial current distributing member is selected which is fabricated of a material which is readily oxidizable or reduced by application of a voltage less than required to hydrolyze water; and
said ionic drug is compounded with a counter-ion which will react with the material of which said current distributing member is fabricated, after said material is oxidised or reduced, to form a neutral compound, said compounded ionic drug being placed into a reservoir through which said drug is permeable.

5. An iontophoresis electrode as claimed in claim 1 further comprising a second charge selective material on the exterior of said reservoir opposite the current distributing member, for contacting the skin when in use and selectively passing ions having the charge of the drug to be delivered.

6. An iontophoresis electrode as claimed in any one of claims 1, 2 and 5, wherein the drug to be delivered is cationic or ionizable into cations, and the charge selective material is selectively permeable to anions.

7. An iontophoresis electrode as claimed in claim 6, wherein the charge selective material applied to the current distributing member comprises an anion exchange membrane.

8. An iontophoresis electrode as claimed in claim 7, wherein the anion exchange membrane is selected from copolymers of styrene and divinylbenzene reacted with trimethylamine.

9. An iontophoresis electrode as claimed in any one of claims 1, 2 and 5, wherein the drug to be delivered is anionic or ionizable into anions, and the charge selective material applied to the current distributing member is selectively permeable to cations.

10. An iontophoresis electrode as claimed in claim 9, wherein the charge selective material applied to the current distributing member comprises a cation exchange membrane.

11. An iontophoresis electrode as claimed in claim 10, wherein the cation exchange membrane is selected from sulphonated styrene polymers and sulphonated fluorocarbon polymers.

12. A method as claimed in claim 3 or claim 4, wherein the drug to be delivered is cationic or ionizable into cations, and the charge selective material is selectively permeable to anions.

13. A method as claimed in claim 12, wherein the charge selective material applied to the current distributing member comprises an anion exchange membrane.

14. A method as claimed in claim 13, wherein the anion exchange membrane is selected from copolymers of styrene and divinylbenzene reacted with trimethylamine.

15. A method as claimed in claim 3 or claim 4, wherein the drug to be delivered is anionic or ionizable into anions, and the charge selective material applied to the current distributing member is selectively permeable to cations.

16. A method as claimed in claim 15, wherein the charge selective material applied to the current distributing member comprises a cation exchange membrane.

17. A method as claimed in claim 16, wherein the cation exchange membrane is selected from sulphonated styrene polymers and sulphonated fluorocarbon polymers.

## Patentansprüche

1. Iontophorese-Elektrode, umfassend:
ein leitfähiges Stromverteilungselement (14);
Mittel (12) zum Koppeln des Stromverteilungselements mit einer elektrischen Stromquelle; und
ein Reservoir (18), das ein zuzuführendes ionisches oder ionisierbares Arzneimittel enthält;
**dadurch gekennzeichne**t, daß eine Schicht (16) aus ladungsselektivem Material an dem Stromverteilungselement zwischen dem Stromverteilungselement und dem Reservoir angebracht ist, wobei das Material für Ionen mit entgegengesetzter Ladung zu der Ladung des Arzneimittels, wenn es ionisiert ist, selektiv ist.

2. Iontophorese-Elektrode nach Anspruch 1, dadurch gekennzeichnet:
das Stromverteilungselement (14) ist aus einem Material hergestellt, das bei einer geringeren Spannung als die zur Hydrolyse von Wasser erforderliche Spannung leicht oxidiert oder reduziert wird; und
das Reservoir (18) ist mit dem Stromverteilungselement gekoppelt und enthält ein ionisches Arzneimittel, das mit einem Gegen-Ion zubereitet ist, das zum Erzeugen einer innerhalb des Reservoirs unlöslichen Verbindung mit dem Material, aus dem das Stromverteilungselement hergestellt ist, reagiert, nachdem das Material oxidiert oder reduziert ist.

3. Verfahren zum Herstellen einer Iontophorese-Elektrode, umfassend die Schritte:
Wählen eines zuzuführenden ionischen oder ionisierbaren Arzneimittels;
Aufnehmen des Arzneimittels innerhalb eines Reservoirs, das für das Arzneimittel durchlässig ist;
Wählen eines leitfähigen Stromverteilungselements;
Wählen eines ladungsselektiven Materials, das für Ionen mit entgegengesetzter Ladung zu der Ladung des Arzneimittels, wenn es ionisiert ist, selektiv durchlässig ist; und
Zusammenbauen der Elektrode durch Anbringen des ladungsselektiven Materials an dem Stromverteilungselement und Befestigen des Stromverteilungselements an dem Reservoir derart, daß das ladungsselektive Material zwischen dem Stromverteilungselement und dem Reservoir angeordnet ist.

4. Verfahren zum Herstellen einer Iontophorese-Elektrode nach Anspruch 3, bei dem:
das zuzuführende gewählte Arzneimittel ist ein ionisches Arzneimittel;
es ist ein Stromverteilungselement gewählt, das aus einem Material hergestellt ist, das bei Anwendung einer kleineren Spannung als zum Hydrolysieren von Wasser erforderlich leicht oxidierbar ist oder leicht reduziert wird; und
das ionische Arzneimittel ist mit einem Gegen-Ion zubereitet, das - um eine neutrale Verbindung zu bilden - mit dem Material, aus dem das Stromverteilungselement hergestellt ist, reagieren wird, nachdem das Material oxidiert oder reduziert ist, wobei das zubereitete ionische Arzneimittel in einem Reservoir angeordnet ist, das für das Arzneimittel durchlässig ist.

5. Iontophorese-Elektrode nach Anspruch 1, ferner umfassend ein zweites ladungsselektives Material an der zu dem Stromverteilungselement entgegengesetzten Außenseite des Reservoirs zur Berührung der Haut im Gebrauch und zum selektiven Durchlassen von Ionen, die die Ladung des zuzuführenden Arzneimittels aufweisen.

6. Iontophorese-Elektrode nach einem der Ansprüche 1, 2 und 5, bei der das zuzuführende Arzneimittel kationisch oder zu Kationen ionisierbar ist und das ladungselektive Material für Anionen selektiv durchlässig ist.

7. Iontophorese-Elektrode nach Anspruch 6, bei der das am Stromverteilungselement angebrachte ladungsselektive Material eine Anionenaustauschmembran umfaßt.

8. Iontophorese-Elektrode nach Anspruch 7, bei der die Anionenaustauschmembran aus Copolymeren von Styrol und Divinylbenzol, umgesetzt mit Trimethylamin, gewählt ist.

9. Iontophorese-Elektrode nach einem der Ansprüche 1, 2 und 5, bei der das zuzuführende Arzneimittel anionisch oder zu Anionen ionisierbar ist und das an dem Stromverteilungselement angebrachte ladungsselektive Material für Kationen selektiv durchlässig ist.

10. Iontophorese-Elektrode nach Anspruch 9, bei der das an dem Stromverteilungselement angebrachte ladungsselektive Material eine Kationenaustauschmembran umfaßt.

11. Iontophorese-Elektrode nach Anspruch 10, bei der die Kationenaustauschmembran aus sulfonierten Styrolpolymeren und sulfonierten Fluorkohlenstoffpolymeren gewählt ist.

12. Verfahren nach Anspruch 3 oder 4, bei dem das zuzuführende Arzneimittel kationisch oder zu Kationen ionisierbar ist und das ladungselektive Material für Anionen selektiv durchlässig ist.

13. Verfahren nach Anspruch 12, bei dem das am Stromverteilungselement angebrachte ladungsselektive Material eine Anionenaustauschmembran umfaßt.

14. Verfahren nach Anspruch 13, bei dem die Anionenaustauschmembran aus Copolymeren von Styrol und Divinylbenzol, umgesetzt mit Trimethylamin, gewählt ist.

15. Verfahren nach Anspruch 3 oder 4, bei dem das zuzuführende Arzneimittel anionisch oder zu Anionen ionisierbar ist und das an dem Stromverteilungselement angebrachte ladungsselektive Material für Kationen selektiv durchlässig ist.

16. Verfahren nach Anspruch 15, bei dem das an dem Stromverteilungselement angebrachte ladungsselektive Material eine Kationenaustauschmembran umfaßt.

17. Verfahren nach Anspruch 16, bei dem die Kationenaustauschmembran aus sulfonierten Styrolpolymeren und sulfonierten Fluorkohlenstoffpolymeren gewählt ist.

## Revendications

1. Électrode d'iontophorèse comprenant:
un élément de distribution du courant, conducteur (14);
des moyens (12) destinés à relier ledit élément de distribution du courant à une source de courant électrique; et
un réservoir (18) renfermant un médicament ionique ou pouvant être ionisé à délivrer;
caractérisée en ce qu'il est prévu une couche (16) de matériau de sélection de charge appliquée sur ledit organe de distribution du courant, qui est entre ledit élément de distribution du courant et ledit réservoir, ledit matériau étant sélectif pour des ions ayant une charge opposée à la charge dudit médicament lorsqu'il est ionisé.

2. Électrode d'iontophorèse selon la revendication 1, caractérisée en ce que:
l'élément de distribution du courant (14) est fabriqué en un matériau qui est facilement oxydé ou réduit à une tension inférieure à la tension requise pour hydrolyser l'eau; et
le réservoir (18) est relié audit élément de distribution du courant et contient un médicament ionique combiné à un contre-ion qui réagit avec ledit matériau à partir duquel ledit élément de distribution du courant est fabriqué, après que ledit matériau soit oxydé ou réduit, pour produire un composé insoluble à l'intérieur dudit réservoir.

3. Procédé de fabrication d'une électrode d'iontophorèse comprenant les étapes consistant à:
sélectionner un médicament ionique ou pouvant être ionisé à délivrer;
enfermer ledit médicament à l'intérieur d'un
réservoir à travers lequel ledit médicament est perméable;
sélectionner un élément de distribution du courant conducteur;
sélectionner un matériau de sélection de charge sélectivement perméable aux ions de charge opposée à celle dudit médicament lorsqu'il est ionisé; et
assembler ladite électrode en appliquant ledit matériau de sélection de charge sur ledit élément de distribution du courant et monter ledit élément de distribution du courant sur ledit réservoir de façon que ledit matériau de sélection de charge soit placé entre ledit élément de distribution du courant et ledit réservoir.

4. Procédé de fabrication d'une électrode d'iontophorèse selon la revendication 3 dans lequel:
le médicament choisi à délivrer est un médicament ionique;
un élément de distribution du courant réactif est sélectionné lequel est fabriqué en un matériau qui peut facilement être ionisé ou réduit par application d'une tension inférieure à celle requise pour hydrolyser l'eau; et
ledit médicament ionique est combiné à un contre-ion qui réagira avec le matériau à partir duquel est fabriqué ledit élément de distribution du courant, après que ledit matériau soit oxydé ou réduit, afin de produire un composé neutre, ledit médicament ionique combiné étant placé dans un réservoir à travers lequel ledit médicament est perméable.

5. Électrode d'iontophorèse selon la revendication 1 comprenant en outre un deuxième matériau de sélection de charge à l'extérieur dudit réservoir opposé à l'élément de distribution du courant, destiné à se mettre en contact avec la peau lors de l'utilisation et à faire passer de manière sélective les ions ayant la charge du médicament à délivrer.

6. Électrode d'iontophorèse selon l'une quelconque des revendications 1, 2 et 5, caractérisé en ce que le médicament à délivrer est cationique ou peut être ionisé en cations, et le matériau de sélection de charge est sélectivement perméable aux anions.

7. Électrode d'iontophorèse selon la revendication 6, caractérisée en ce que le matériau de sélection de charge appliqué sur l'élément de distribution du courant comprend une membrane échangeuse d'anions.

8. Électrode d'iontophorèse selon la revendication 7, caractérisée en ce que la membrane échangeuse d'anions est choisie parmi des copolymères de styrène et de butadiène benzène en réaction avec de la triméthylamine.

9. Électrode d'iontophorèse selon l'une quelconque des revendications 1, 2 et 5, caractérisée en ce que le médicament à délivrer est anionique ou peut être ionisé en anions, et le matériau de sélection de charge appliqué sur l'élément de distribution du courant est sélectivement perméable aux cations.

10. Électrode d'iontophorèse selon la revendication 9, caractérisée en ce que le matériau de sélection de charge appliqué sur l'élément de distribution du courant comprend une membrane échangeuse de cations.

11. Électrode d'iontophorèse selon la revendication 10, caractérisée en ce que la membrane échangeuse de cations est choisie parmi des polymères de styrène sulfoné et des polymères de fluorocarbone sulfoné.

12. Procédé selon la revendication 3 ou la revendication 4, caractérisé en ce que le médicament à délivrer est cationique ou peut être ionisé en cations, et le matériau de sélection de charge est sélectivement perméable aux anions.

13. Procédé selon la revendication 12, caractérisé en ce que le matériau de sélection de charge appliqué sur l'élément de distribution du courant comprend une membrane échangeuse d'anions.

14. Procédé selon la revendication 13, caractérisé en ce que le membrane échangeuse d'anions est choisie parmi des copolymères de styrène et de butadiène benzène en réaction avec de la triméthylamine.

15. Procédé selon la revendication 3 ou la revendication 4, caractérisé en ce que le médicament à délivrer est anionique ou peut être ionisé en anions, et le matériau de sélection de charges appliqué sur l'élément de distribution du courant est sélectivement perméable aux cations.

16. Procédé selon la revendication 15, caractérisé en ce que le matériau de sélection de charge appliqué sur l'élément de distribution du courant comprend une membrane échangeuse de cations.

17. Procédé selon la revendication 16, caractérisé en ce que la membrane échangeuse de cations est choisie parmi des polymères de styrène sulfoné et des polymères de fluorocarbone sulfoné.
